(19) Europäisches Patentamt
European Patent Office
Office européen des brevets

(11) **EP 2 147 674 A1**

(12) **EUROPEAN PATENT APPLICATION**

(43) Date of publication:
**27.01.2010 Bulletin 2010/04**

(21) Application number: **08305421.3**

(22) Date of filing: **24.07.2008**

(51) Int Cl.:
*A61K 31/58* (2006.01)   *A61P 17/04* (2006.01)
*A61P 35/00* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Besins Healthcare**
**1050 Bruxelles (BE)**

(72) Inventor: **Masini, Valérie**
**92340 Bourg La Reine (FR)**

(74) Representative: **Cabinet Plasseraud**
**52, rue de la Victoire**
**75440 Paris Cedex 09 (FR)**

(54) **Transdermal pharmaceutical compositions comprising danazol**

(57)    The present invention relates to transdermal danazol-containing pharmaceutical compositions, such as gels, and to methods of making and using the same.

## Description

### FIELD OF THE INVENTION

The present invention relates to transdermal danazol-containing pharmaceutical compositions, such as gels, and to methods of making and using the same.

### BACKGROUND

The present invention relates to danazol-containing pharmaceutical compositions, such as gels, and to methods of making and using the same.

The compound danazol or (17α)-Pregna-2,4-dien-20-yno[2,3-d]isoxazol-17β-ol, is a derivative of the synthetic steroid ethisterone, a modified testosterone. It is classified as an antigonadotropin, since it decreases the levels of the pituitary hormones follicle-stimulating hormone (FSH) and luteinizing hormone (LH).

Danazol has been commercialized, e.g. under the trade names Danatrol® and Danocrine®, for oral administration. The initial indication of this drug was for use in the treatment of endometriosis. Later, it was demonstrated that danazol can also be used for the treatment of breast diseases, including breast pain and gynecomastia, and for the treatment of skin disorders. However, there has been observed a low compliance of patients with such oral treatments with danazol (Wong A.Y., Tang L., Fertil Steril 2004; 81(6), 1522-7). Indeed, at the typically prescribed oral dosage (100 mg to 400 mg per day), a high drop-out rate occurs, most likely because of adverse side effects, particularly androgenic side effects, such as growth of facial hair, acne, weight gain, dandruff and deepening of the voice. Systemic administration of danazol may increase the risk for unhealthy cholesterol levels. Pregnant women or those trying to become pregnant should not take this drug because it may cause birth defects. A few cases of blood clots and strokes have been reported, as well as rare cases of liver damage.

Danazol is indeed significantly metabolized upon first liver passage. Moreover, its metabolites are not active, thereby significant amount of orally administered danazol are metabolized into inactive products before reaching their final target.

Document WO2004/060322 to FemmePharma relates to formulations for topical or local administration of drugs directly to the breast or chest, for treatments of diseases and disorders of the breast. This document discloses a drug formulation comprising danazol in a formulation which contains 95 to 100% propylene glycol, which is generally considered to be an irritating and/or sensitizing amount of propylene glycol.

There remains a need, therefore, for pharmaceutical compositions suitable for non-oral administration of danazol.

### SUMMARY

The present invention provides a pharmaceutical composition for topical administration to a skin surface wherein said composition comprises:

(i) a therapeutically effective amount of danazol,
(ii) at least one monoalcohol,
(iii) optionally, a non-irritating and/or non-sensitizing amount of at least one penetration enhancer,
(iv) optionally, at least one gelling agent,
(v) optionally, at least one moisturizer,
(vi) an aqueous vehicle.

In one embodiment, said composition comprises:

(i) 0.01 1 to 10 % (w/w) of danazol,
(ii) 10 to 90 % (w/w) of at least one monoalcohol,
(iii) 0 to 10 % (w/w) of at least one penetration enhancer,
(iv) 0 to 5 % (w/w) of at least one gelling agent,
(v) 0 to 30 % (w/w) of at least one moisturizer,
(vi) q.s. 100 % (w/w) water.

Said monoalcohol may be selected from the group consisting of ethanol and isopropanol.
Said penetration enhancer may be selected from the group consisting of isopropyl myristate and propylene glycol.

Said gelling agent may be selected from the group consisting of polyacrylic acids, cellulosics, and mixtures thereof.

Said moisturized may comprise glycerine.

The invention also provides a pharmaceutical composition as described above and elsewhere herein for treating a patient suffering from or at risk of developing a breast disorder selected from benign breast diseases, gynecomastia, breast cancer, mastalgia and conditions involving dense breast tissue.

The invention also provides a pharmaceutical composition as described above and elsewhere herein for treating a patient suffering from or at risk of developing a skin disorder selected from pruritus, erythema, urticaria, and dermatitis.

The invention also provides the use ofdanazol in the preparation of a pharmaceutical composition as described above and elsewhere herein, wherein said pharmaceutical composition is for treating a patient suffering from or at risk of developing a breast disorder selected from benign breast diseases, gynecomastia, breast cancer, mastalgia and conditions involving dense breast tissue.

The invention also provides the use of danazol in the preparation of a pharmaceutical composition as described above and elsewhere herein, wherein said pharmaceutical composition is for treating a patient suffering from or at risk of developing a skin disorder selected from pruritus, erythema, urticaria, and dermatitis.

The invention also provides a dose packet, unit dose packet or multiple dose packet containing a pharmaceutical composition as described above and elsewhere herein.

The invention also provides a dispenser, optionally with hand pump, containing a pharmaceutical composition as described above and elsewhere herein.

The invention also provides a device comprising:

(A) a reservoir containing a pharmaceutical composition comprising:

(i) a therapeutically effective amount of danazol,
(ii) at least one 1-nonoalcohol,
(iii) optionally, a non-irritating and/or non sensitizing amount of at least one penetration enhancer,
(iv) optionally, at least one gelling agent,
(v) optionally, at least one moisturizer, and

(B) a topical applicator.

In one embodiment, there is a provided a process for preparing a pharmaceutical composition as described above and elsewhere herein comprising the steps of preparing a mixture comprising:

(i) at least one monoalcohol
(ii) danazol,
(iii) an aqueous vehicle,
(iv) optionally, a non-irritating and/or non-sensitizing amount of at least one penetration enhancer,
(v) optionally, at least one gelling agent,
(vi) optionally, at least one moisturizer.

The invention provides a method of administering a therapeutically effective amount of danazol to a patient in need thereof, comprising topically administering to a surface of skin of said patient a pharmaceutical composition as described above and elsewhere herein.

The invention provides a method for treating a patient suffering from or at risk of developing a breast disorder comprising topically administering to a surface of skin of a patient in need thereof a therapeutically effective amount of a pharmaceutical composition as described above and elsewhere herein.

The invention also provides a method for treating a patient suffering from or at risk of developing a skin disorder comprising topically administering to a surface of skin of a patient in need thereof a therapeutically effective amount of a pharmaceutical composition as described above and elsewhere herein.

## DETAILED DESCRIPTION

For the purposes of this disclosure and unless otherwise specified, "a" or "an" means "one or more."

In accordance with one aspect, the invention provides a pharmaceutical composition comprising danazol. In some embodiments, the composition is suitable for transdermal or transcutaneous delivery.

Formulating drugs for percutaneous delivery is a difficult and unpredictable art. In particular, the formulation of hormones, steroids, and steroid-like compounds for percutaneous delivery has proven delicate as each active ingredient tends to behave differently in terms of transdermal flux for example.

The compositions described herein were surprisingly and unexpectedly found to yield good results in *in vitro* experiments measuring transdermal flux of danazol in Franz cells, as illustrated in Example 4. As described in more detail below, *in silico* predictions for transdermal flux of danazol were carried out. Due to the close structural relationship of danazol to testosterone, these predictions were compared to equivalent predictions made for testosterone. (Testosterone also is a good reference compound because there are a number of commercially successful pharmaceutical testosterone gels for transdermal application.) The results of this analysis predicted that the permeation rate (flux) for danazol would be almost 10 times lower than that for testosterone. In view of the low predicted permeation rate of danazol, it was surprising and unexpected that the compositions described herein achieve satisfactory flux, including flux that is comparable to that observed with testosterone.

In one embodiment, the pharmaceutical composition comprises:

(i) a therapeutically effective amount of danazol,
(ii) at least one monoalcohol,
(iii) optionally, a non-irritating and/or non-sensitizing amount of at least one penetration enhancer,
(iv) optionally, at least one gelling agent,
(v) optionally, at least one moisturizer,
(vi) an aqueous vehicle.

In some embodiments, the compositions according to the invention do not require any adhesive, matrix or membrane for administration, by contrast to patches or occlusive systems. Such embodiments offer clear advantages over known compositions that require an adhesive, such as avoiding the use of potentially irritating ingredients.

The compositions of the invention offer further advantages, including being non-irritating to the skin and resulting in limited side effects. The compositions of the invention allow a local, non systemic delivery of danazol and result in a local effect of the drug which avoids systemic side effects, such as gastrointestinal irritation or effects attributable to androgenic activity, such as acne, oily skin or hair, hirsutism, weight gain, deepening of the voice, and androgenic alopecia.

As a result of these and other advantages, the compositions facilitate patient compliance.

## Compositions

As noted above, in one aspect a pharmaceutical composition of the present invention comprises:

(i) a therapeutically effective amount of danazol,
(ii) at least one monoalcohol,
(iii) optionally, a non-irritating and/or non-sensitizing amount of at least one penetration enhancer,
(iv) optionally, at least one gelling agent,
(v) optionally, at least one moisturizer,
(vi) an aqueous vehicle.

In some embodiments, the composition comprises the specified components. In some embodiments, the composition consists of the specified components. In other embodiments, the composition consists essentially of the specified components. As used herein, "consists essentially of" the specified components means that the composition includes at least the specified components, and may also include other components that do not materially affect the basic and novel characteristics of the invention.

As noted above, compositions of the invention are suitable for transdermal administration. For example, the compositions can be directly applied to a surface of the skin, for direct non-occlusive transdermal/transcutaneous application. As used herein, the terms "direct"/"directly" and "non-occlusive" reflect that the compositions of the invention do not require a matrix or membrane to effect administration, and thus are not required to be dispensed via a patch, plaster, tape system, or the like. However, the compositions of the invention can be dispensed via a patch, plaster, tape system or the like.

In some embodiments, the amount of composition administered is a defined, finite amount that provides a therapeutically effective amount (e.g., a single daily dose) of danazol.

As used herein, the phrase "therapeutically effective amount" means an amount (dosage) that achieves the specific pharmacological response for which the drug is administered in a given patient. It is emphasized that a "therapeutically effective amount" of a drug that is administered to a particular subject in a particular instance may not always be effective in treating the target conditions/diseases, even though such dosage is deemed to be a therapeutically effective amount by those of skill in the art. Those skilled in the art will recognize that the "therapeutically effective amount" may vary from patient to patient, or from condition to condition, and can determine a "therapeutically effective amount" for a given patient/condition by routine means.

In some embodiments, the composition is administered to a surface of the skin over a defined surface area. The administration of a defined, finite amount of the composition to a defined surface area permits the control of the amount of active principal, i.e., danazol that is applied to a given surface area, i.e., controlling the local concentration. By controlling (e.g., limiting) local concentration, local side effects, such as local androgenic effects (including, but not limited to: acne, oily skin), can be minimized.

**Danazol**

As noted above, the compositions of the invention comprise a therapeutically effective amount of danazol.

As used herein, the term "danazol" refers to $(17\alpha)$-Pregna-2,4-dien-20-yno[2,3-d]isoxazol-17$\beta$-ol. Those skilled in the art will understand that pharmaceutically acceptable active enantiomers, isomers, tautomers, salts, chelates, esters, amides and derivatives of danazol also can be used.

Typically, the composition of the invention comprises 0.01 to 10 % (w/w) of danazol, for example 0.05 to 5% (w/w), and for example 0.5 to 1.5 % (w/w) of danazol. Typically, the composition of the invention may comprise 0.05 %, 0.1 %, 0.2 %, 0.3 %, 0.4 %, 0.5 %, 0.6 %, 0.7 %, 0.8 %, 0.9, % 1 %, 1.5 %, or 2% (w/w) of danazol.

Unless otherwise stated, percentages (%) refer to amounts by weight based upon total weight of the composition (w/w).

**Monoalcohols**

As noted above, the compositions of the invention comprise at least one monoalcohol. As used herein the term "monoalcohol" refers to an organic molecule containing at least one carbon atom and one only alcohol group -OH.

Exemplary monoalcohols are C2-C6 monoalcohols and can include C2-C4 alcohols, such as ethanol, n-propanol, isopropanol, n-butanol, isobutanol, tert-butanol, or mixtures thereof. Exemplary monoalcohols suitable for the compositions of the invention are ethanol and isopropanol.

The presence of such a monoalcohol can act as a solvent for danazol. Typically, the greater the amount of danazol in the composition, the more monoalcohol will be used to solubilize said danazol.

The presence of such a monoalcohol may also contribute to accelerate drying of the composition onto the skin.

Typically, the monoalcohol will be used in an amount between 10 % and 90 % (w/w) of the total composition, for example in an amount of 10%, 20%, 30%, 40%, 50%, 60%, 70%, 75%, 80%, 85% or 90% of the total composition.

**Penetration enhancer**

A "penetration enhancer" is an agent known to accelerate the delivery of the drug through the skin. These agents can also be referred to as accelerants, adjuvants, absorption promoters, and are collectively referred to herein as "enhancers". This class of agents includes those with diverse mechanisms of actions including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin such as changing the state or fluidity of the stratum corneum intercellular lipid bilayers.

In one embodiment, the penetration enhancer of the present invention is a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms. Non-limiting examples of penetration enhancers include C8-C22 fatty acids such as isostearic acid, octanoic acid, and oleic acid; C8-C22 fatty alcohols such as oleyl alcohol and lauryl alcohol; lower alkyl esters of C8-C22 fatty acids such as ethyl oleate, isopropyl myristate, butyl stearate, and ethyl laurate; di(lower)alkyl esters of C6-C8 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; Nalkylpyrrolidone; and terpenes.

Suitable penetration enhancers include urea, oleic acid, isopropyl myristate and propylene glycol.

As used herein, the expression "non-irritating and/or non-sensitizing amount of at least one penetration enhancer" refers to amounts which the skilled person in the art would consider to be well-tolerated by the human skin, i.e., dermatologically acceptable. Using common general knowledge, the skilled person can determine non-irritating and/or non sensitizing amounts of penetration enhancer. In some embodiments, the non-irritating and/or non-sensitizing amount results in no detectable or sustained dermal adverse reaction (e.g., itching, reddening, burning sensation), or results in only a minimal reaction that is generally deemed to be acceptable by patients and health care providers.

Typically, the penetration enhancer will be used in an amount which does not exceed 10 % (w/w) of the total composition, for example no more than 9%, 8%, 7%, 6%, and for example no more than 5%, 4%, 3%, 2%, 1%, 0.5 % (w/w) of the total composition.

In one embodiment of the invention, the penetration enhancer will be used in an amount of at least 0.01%, 0.02%, 0.03%, 0.04%, 0.05%, 0.1%, 0.2% (w/w) of the total composition.

In one embodiment of the invention, the penetration enhancer will be used in amount between 0.01% and 10% (w/w) of the composition, for example in an amount between 0.02% and 7%, between 0.05% and 5%, between 0.1% and 1% (w/w) of the total composition.

**Gelling Agents**

As noted above, the compositions of the invention may optionally comprise at least one gelling agent.

As used herein, the term "gelling agent" specifies a compound, optionally of polymeric nature, having the capacity to form a gel when contacted with a specific solvent, e.g., water. Gelling agents (e.g., thickeners) are known in the art. Gelling agents may act to increase the viscosity of the pharmaceutical compositions of the invention. For example, a gelling agent may provide the composition with sufficient viscosity to allow easy application of the composition onto the skin. Additionally or alternatively, gelling agents may act as solubilizing agents.

Examples of gelling agents include anionic polymers such as acrylic acid based polymers (including polyacrylic acid polymers, e.g. CARBOPOL® by Noveon, Ohio), cellulose derivatives, poloxamers and poloxamines, more precisely, Carbomers which are acrylic acid-based polymers, e.g. Carbopol® 980 or 940, 981 or 941, 1342 or 1382, 5984, 934 or 934P (Carbopol® are usually polymers of acrylic acid crosslinked with allyl sucrose or allylpentaerythritol), Ultrez, Pemulen TR1® or TR2®, Synthalen CR, etc.; cellulose derivatives such as carboxymethylcelluloses, hydroxypropylcelluloses (Klucel®), hydroxyethylcelluloses, ethylcelluloses, hydroxymethylcelluloses, hydroxypropylmethylcelluloses, and the like, and mixtures thereof; poloxamers or polyethylene-polypropylene copolymers such as Lutrol ® grade 68 or 127, poloxamines and other gelling agents such as chitosan, dextran, pectins, and natural gums. Any one or more of these gelling agents may be used alone or in combination in the pharmaceutical compositions according to the invention. In one aspect, the gelling agent is selected from the group consisting of polyacrylic acids, cellulosics, and mixtures thereof.

Typically, the gelling agent will be used in an amount which does not exceed 5% (w/w) of the total composition, for example no more than 4%, 3%, 2%, 1%, 0.5 % (w/w) of the total composition.

In one embodiment of the invention, the gelling agent will be used in an amount of at least 0.01%, 0.02%, 0.03%, 0.04%, 0.05%, 0.1%, 0.2% (w/w) of the total composition.

In one embodiment of the invention, the gelling agent will be used in amount between 0.01% and 5% (w/w) of the composition, for example in an amount between 0.02% and 3%, between 0.05% and 2%, between 0.1% and 1% (w/w) of the total composition.

**Moisturizers**

As noted above, the compositions of the invention may optionally comprise at least one moisturizer.

As used herein "moisturizer" specifies an agent that hydrates the skin. Moisturizers are known in the art. Moisturizers can be used either alone or in combination, e.g., a combination of two or three (or more) different moisturizers can be used. In some embodiments, moisturizers are selected from emollients and/or humectants.

As used herein, "emollients" specify substances that soften the skin and tend to improve moisturization of the skin. Emollients are well known in the art, and include mineral oil, petrolatum, polydecene, isohexadecane, fatty acids and alcohols having from 10 to 30 carbon atoms; pelargonic, lauric, myristic, palmitic, stearic, isostearic, hydroxystearic, oleic, linoleic, ricinoleic, arachidic, behenic, and euricic acids and alcohols; triglyceride esters, castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, acetoglyceride esters, ethoxylated glycerides, ethoxylated glyceryl monostearate, alkyl esters of fatty acids having 10 to 20 carbon atoms, hexyl laurate, isohexyl laurate, isohexyl palmitate, isopropyl palmitate, decyl oleate, isodecyl oleate, hexadecyl stearate, decyl stearate, diisopropyl adipate, diisohexyl adipate, diisopropyl sebacate, laurly lactate, myristyl lactate, acetyl lactate; alkenyl esters of fatty acids having 10 to 20 carbon atoms, oleyl myristate, oleyl stearate, oleyl oleate, fatty acid esters of ethoxylated fatty alcohols, polyhydric alcohol esters, ethylene glycol mono and di-fatty acid esters, diethylene glycol mono- and di-fatty acid esters, polyethylene glycol, wax esters, beeswax, spermaceti, myristyl myristate, stearyl stearate, silicone oils, dimethicones, cyclomethicones. In some embodiments, the composition comprises one or more emollients that are liquid at room temperature.

As used herein "humectants" specifies hygroscopic substances that absorb water from the air. Humectants suitable for use in the invention include glycerine, propylene glycol, glyceryl triacetate, a polyol, sorbitol, maltitol, a polymeric polyol, polydextrose, quillaia, lactic acid, and urea.

Moisturizers suitable for use in the present invention may comprise amines, alcohols, glycols, amides, sulfoxides, and pyrrolidones. In one aspect, the moisturizer is selected from the group consisting of lactic acid, glycerine, propylene glycol, and urea.

Typically, the moisturizer will be used in an amount which does not exceed 30 % (w/w) of the total composition, for example no more than 20%, and for example no more than 15%, 10%, 8%, 7%, 5% (w/w) of the total composition.

In one embodiment of the invention, the moisturizer will be used in an amount of at least 0,01%, 0.02%, 0.03%, 0.04%, 0.05%, 0.1%, 0.2%, 0.5% (w/w) of the total composition.

In one embodiment of the invention, the moisturizer will be used in amount between 0.01% and 30% (w/w) of the composition for example in an amount between 0.05% and 20%, between 0.1% and 10%, between 0.5% and 5% (w/w) of the total composition.

In one embodiment, the composition comprises between 0.01% and 30% (w/w) of glycerine, for example between 0.05% and 20%, between 0.1% and 10%, between 0.5% and 5% (w/w) of glycerine.

**Aqueous vehicle**

As noted above, the composition of the invention comprises an aqueous vehicle.

Aqueous vehicles are known in the art. According to one aspect of the invention, the aqueous vehicle comprises, besides water, ingredients useful in adjusting the pH, for instance at least one buffering agent. Buffering agents, especially pharmaceutically acceptable buffering agents, are known in the art. In one aspect, said aqueous vehicle comprises at least one buffer, typically selected from the group consisting of citrate buffers, e.g., sodium citrate and/or potassium citrate; tris buffers, e.g., tris maleate; phosphate buffers, including Sorensen-type buffers, dibasic or monobasic phosphate, e.g., sodium dibasic or monobasic phosphate.

In another aspect, the pharmaceutical composition of the invention further comprises a base. Advantageously, said base is for example pharmaceutically acceptable, and is typically selected from the group consisting of triethanolamine, sodium hydroxide, ammonium hydroxide, potassium hydroxide, arginine, aminomethylpropanol or tromethamine, and mixtures thereof. Where the pH of said pharmaceutical composition is not optimized for transdermal administration, e.g., where the gelling agent comprises at least one acrylic acid-based polymer, the use of a base contributes to the neutralization of said pharmaceutical composition, for topical administration onto a skin surface. Furthermore, the use of said base (neutralizer) may allow for optimum swelling of the polymer chains during the neutralization of the charges and the formation of polymer salts. In embodiments where said gelling agent comprises acrylic acid-based polymer, the base typically comprises triethanolamine. The use of a base also may allow optimal viscosity to be achieved. The skilled person will know how to choose a suitable amount of base for use in the composition, and may select the base based on the nature of the gelling agent present therein, and the alcohol content of the composition. For example, with carbomers and/or if there is a high alcohol content, one can use tromethamine and/or NaOH as a base, in amounts chosen so as to reach the desired final pH in the composition.

**Further Optional Components**

The pharmaceutical compositions of the invention optionally may comprise other usual pharmaceutical additives, including salt(s), stabilizer(s), antimierobial(s) such as paraben compounds, fragrance(s), and/or propellant(s).

Depending on the nature of the selected ingredients, it may be advantageous to include a surfactant. Surfactants are known in the art, and the skilled person can select suitable surfactants for use in the present invention, such as surfactants that are dermatologically and/or cosmetically acceptable.

Examples thereof include non-ionic surfactants, for example:

- esters, such as:

  ○ esters of polyethyleneglycol and fatty acids, including Labrasol®, which is a mixture of mono, di and triglycerides and of mono and diesters of polyethyleneglycol and fatty acids;
  ○ esters of saccharose and fatty acids, such as: sucrose laurate with HLBJ6; sucrose palmitate with HLB 16;
  ○ esters of sorbitanne polyoxyethylene, such as Tween® compounds including Tween® 20, 60 and/or 80;

- alkylene oxide copolymers, such as copolymers of ethylene oxide and propylene oxide, e.g. Pluronics®.

Further examples include anionic surfactants such as SDS (sodium dodecyl sulphate), and the like and cationic surfactants such as cetrimide (Alkyltrimethylammonium bromide) and the like.

Typically, surfactants will be used in the compositions of the invention in an amount which does not exceed 5% (w/w), for example no more than 4%, 3%, 2%, 1%, 0.5% of the total composition.

In one embodiment of the invention, surfactants will be used in the compositions of the invention in an amount of at least 0.01%, for example at least 0.02%, 0.05%, 0.1% of the total composition.

**Exemplary Compositions**

Exemplary, non-limiting compositions are provided below. As mentioned above, percentages (%) refer to amounts by weight based upon total weight of the composition (w/w). The sum of the different components of the composition adds up to 100% (w/w) of the total composition.

In one aspect, the present invention relates to a pharmaceutical composition for topical administration to a skin surface wherein said composition comprises:

(i) 0.01 to 10 % (w/w) of danazol,
(ii) 10 to 90 % (w/w) of at least one monoalcohol, e.g., ethanol or isopropanol,
(iii) 0 to 10 % (w/w) of at least one penetration enhancer, e.g., isopropyl myristate or propylene glycol,
(iv) 0 to 5 % (w/w) of at least one gelling agent, e.g., polyacrylic acids, cellulosics, or mixtures thereof,
(v) 0 to 30 % (w/w) of at least one moisturizer, e.g., glycerine,
(vi) q.s. 100 % (w/w) water.

In another aspect, the present invention relates to a pharmaceutical composition for topical administration to a skin surface wherein said composition comprises:

(i) 0.02 to 5 % (w/w) of danazol,
(ii) 20 to 80 % (w/w) of at least one monoalcohol, e.g., ethanol or isopropanol,
(iii) 0 to 8 % (w/w), for example 0.01 to 7 % (w/w), and for example 0.05 % to 5% (w/w), of at least one penetration enhancer, e.g., isopropyl myristate or propylene glycol,
(iv) 0 to 4 % (w/w), for example 0.01 to 3% (w/w), and for example 0.05 to 2% (w/w), of at least one gelling agent, e.g., polyacrylic acids, cellulosics, or mixtures thereof,
(v) 0 to 20 % (w/w), for example 0.01 to 10%, and for example 0.05 to 5% (w/w), of at least one moisturizer, e.g., glycerine,
(vi) q.s. 100 % (w/w) water.

In another aspect, the present invention relates to a pharmaceutical composition for topical administration to a skin surface wherein said composition comprises:

(i) 0.05 to 2.5 % (w/w) of danazol,
(ii) 30 to 70 % (w/w) of at least one monoalcohol, e.g., ethanol or isopropanol,
(iii) 0 to 5 % (w/w), for example 0.01 to 4%, and for example 0.05 to 3% (w/w), of at least one penetration enhancer, e.g., isopropyl myristate or propylene glycol,
(iv) 0 to 2 % (w/w), for example 0.01 to 1.5%, and for example 0.05 to 1%, of at least one gelling agent, e.g., polyacrylic acids, cellulosics, or mixtures thereof,
(v) 0 to 10 % (w/w), for example 0.01 to 5%, and for example 0.05 to 3% (w/w), of at least one moisturizer, e.g., glycerine,
(vi) q.s. 100 % (w/w) water.

In another aspect, the present invention relates to a pharmaceutical composition for topical administration to a skin surface wherein said composition comprises:

(i) 1 % (w/w) of danazol,
(ii) 50 to 70 % (w/w) of at least one monoalcohol, e.g., ethanol or isopropanol,
(iii) 0 to 1 % (w/w), for example 0.01 to 0.7%, and for example 0.05 to 0.5% (w/w), of at least one penetration enhancer, e.g., isopropyl myristate or propylene glycol,
(iv) 0 to 1 % (w/w), for example 0.01 to 0.7%, and for example 0.05 to 0.5% (w/w), of at least one gelling agent, e.g., polyacrylic acids, cellulosics, or mixtures thereof,
(v) 0 to 5 % (w/w), for example 0.01 to 4%, and for example 0.05 to 3% (w/w), of at least one moisturizer, e.g., glycerine,
(vi) q.s. 100 % (w/w) water.

In one embodiment, the composition of the invention comprises:

- 1% (w/w) of danazol,
- 0.9 % (w/w) of Carbopol 980 (neutralized with 0. 1 N NaOH),
- 0.5 % (w/w) of isopropyl myristate,
- 67 % (w/w) of ethanol,
- q.s. 100 % (w/w) of water.

In one embodiment, the composition of the invention comprises:

- 0.84 % (w/w) of danazol,
- 67% (w/w) of ethanol,
- q.s. 100% (w/w) of water.

In one embodiment, the composition of the invention comprises:

- 2% (w/w) of danazol,
- 67% (w/w) of ethanol
- 3% (w/w) cetrimide,
- q.s. 100 % (w/w) of water.

**Exemplary Modes of Administration**

The compositions may be administered by any means effective to apply the composition to a surface of the skin. For example, the compositions may be applied manually, with an applicator such as a dropper or pipette, an applicator such as a swab, brush, cloth, pad, sponge or with any other applicator, such as a solid support comprising paper, cardboard or a laminate material, including material comprising flocked, glued or otherwise fixed fibers. Alternatively, the compositions may be applied as an aerosol or non-aerosol spray, from a pressurized or non-pressurized container. In some embodiments, the compositions are administered in metered doses, such as from a metered dose applicator or from an applicator comprising a single dose of the composition.

**Devices**

One aspect of the invention provides a device for administering the compositions. In one embodiment, the device comprises a reservoir containing the composition and a topical applicator for applying the composition to a surface of the skin.

In one embodiment, the device is an opaque device which protects the composition of the invention from the adverse effects of light. Indeed, danazol is a light-sensitive compound which deteriorates upon prolonged exposure to light.

The reservoir may be of any configuration and any material suitable for containing the composition. For example, the reservoir may be rigid or flexible, may be of a unitary construction (such as a molded material) or may be formed from different pieces secured together, such as by laminating, heat-sealing, gluing, welding, riveting, etc. For example, the reservoir may comprise a rolled wall, two walls substantially parallel joined at the vicinity of their periphery (where the walls may be, for example, flexible/deformable, formed by a thermoformed blister, or rigid), or a bottom wall and a cylindrical wall, or any other configuration suitable for containing the composition. In some embodiments, the reservoir comprises a bag, a pouch, a sachet, a blister, an ampoule, a pipette, a vial, a canister, or a bottle. In some embodiments, the reservoir comprises a deformable wall that is adapted to actuate flow of the composition when deformed such as those described in patent applications WO9527569, WO9615045 or US5630531. Deformable airtight reservoirs provide a good security against leakage of the composition and enable improved preservation of the composition, by protecting it from any atmospheric contact. In some embodiments, the reservoir is adapted to contain a single dose of the composition.

As used herein "topical applicator" specifies an applicator of any configuration and any material suitable for applying the composition to a surface of the skin. The topical applicator may be integrally formed with the reservoir, such that the reservoir and topical applicator comprise a unitary construction, or the topical applicator may be detachable from, or provided separately from, the reservoir.

For example, the topical applicator may comprise a dropper, pipette, swab, brush, cloth, pad, sponge, or any solid support, such as a support comprising paper, cardboard or a laminate material, including material comprising flocked, glued or otherwise fixed fibers. In some embodiments, the applicator is pre-loaded with composition, for example, the applicator may be impregnated with composition, such as with a unit dose of the composition. In other embodiments, the applicator is loaded with composition during use.

Alternatively, the topical applicator may comprise an aerosol or non-aerosol spray device, such as a hand pump.

In other embodiments, the topical applicator is an opening that permits the product to be dispensed therethrough. In some embodiments, the opening is provided with a removable and replaceable device for closing and opening the opening, such as a cap, stopper or plug, which can be placed within or over the opening such as by insertion, screwing, snapping, fitting, or otherwise. In another embodiment, the opening is provided with a removable and disposable device for opening the opening, such as any removable or secable, frangible, peelable or tearable covering over the opening. In other embodiments, the opening is provided with a nozzle or valve, such as a metered dose valve.

In some embodiments, the topical applicator is adapted to dispense a metered dose of the composition, such as a unit dose of a therapeutically effective amount of the composition. In some embodiments, the topical applicator is not a syringe, and the device does not comprise a syringe for intravenous administration.

In some embodiments, the device comprises a single reservoir. In other embodiments, the device contains two or more reservoirs, where each reservoir may contain a single dose of the composition, or may contain any amount of the composition. In some embodiments, the device comprises a single applicator for applying composition from two or more reservoirs. In other embodiments, the device comprises one applicator for applying composition from each reservoir.

In some embodiments, the invention provides a dose, unit dose, or multiple dose of the pharmaceutical composition, such as in a dose package, a unit dose package or a multiple dose package. In some embodiments, the packaging reflects a dosing regimen or schedule of application, e.g. daily, weekly, or twice weekly administration. Advantageously, such packaging of the pharmaceutical composition facilitates accurate application of an amount of the composition, such as a therapeutically effective amount.

According to one embodiment, the composition, device or packet is provided together with instructions for the use thereof in accordance with the methods described herein.

**Therapeutic Methods**

The present invention also provides a method for treating a patient suffering from or at risk of developing a breast disorder, comprising administering a therapeutically effective amount of a pharmaceutical composition according to the invention. The present invention also provides a method for treating a patient suffering from or at risk of developing a skin disorder, comprising administering a therapeutically effective amount of a pharmaceutical composition according to the invention.

The term 'treat' or 'treating' or 'treatment' as used herein refers to the administration of a therapeutically effective amount of a pharmaceutical composition according to the invention to a mammalian subject suffering from or at risk of developing a condition, disorder, or disease. In accordance with some aspects of the invention, the administration may result in preventing the condition, disorder, or disease from occurring to a clinically diagnosable extent in a patient who may be predisposed to the condition, disorder, or disease, but not yet diagnosed as having the condition, disorder, or disease. In accordance with other aspects, the administration may result in inhibiting the condition, disorder, or disease, for example, arresting the development of the condition, disorder, or disease, relieving the condition, disorder, or disease, for example, causing regression of the condition, disorder, or disease, or relieving a condition caused by the disease or disorder, for example, stopping or reducing a symptom of the disease or disorder. Any such result may constitute the achievement of an intended therapeutic effect in a patient.

In one embodiment, the administration is performed by applying a therapeutically effective amount of the composition of the invention onto a surface of the skin of a patient in need thereof. In some embodiments, the patient to be treated is a mammal, such as a human. The patient may be a male or a female.

In some embodiments, the administration further comprises rubbing the composition into the patient's skin. This rubbing may comprise, for example, gentle rubbing of the composition onto the selected surface area, so that the composition substantially completely penetrates into the patient's skin.

The administration may follow any suitable administration regimen, as can be determined by those skilled in the art. For example, in one aspect, the method of the invention comprises once daily administration. In another aspect, the method comprises bi-weekly or once-weekly administration. Other suitable regimens are included within the scope of the invention.

The present invention also relates to the use of one of the above compositions for the manufacture of a medicament for treating a patient suffering from or at risk of developing a breast disorder. The present invention also relates to the use of one of the above compositions for the manufacture of a medicament or for treating a patient suffering from or at risk of developing a skin disorder.

In particular, the pharmaceutical compositions, gels, packets and containers of the invention are useful for:

-treating a patient suffering from or at risk of developing a breast disorder such as:

- conditions involving dense breast tissue. High density breast tissues are a predictor of breast cancer risk, and compromises mammographic sensitivity, which is a major issue for cancer detection and diagnostic. Said dense breast tissue can be diffuse or nodular;

- benign breast diseases. Benign breast disease generally refers to a constellation of common non-malignant aberrations in breast tissue. These aberrations include numerous lesions that have well-defined histological characteristics, and can be classified as proliferative or nonproliferative. Exemplary benign breast diseases treatable by the present methods include adenosis, cysts, duct ectasia, fibroadenoma, fibrosis, hyperplasia, metaplasia and other fibrocystic changes. Each of these diseases, often referred to as "changes" or "conditions" due to their prevalence, have well-defined histological and clinical characteristics. "Adenosis" refers to generalized glandular disease of the breast. It typically involves an enlargement of breast lobules, which contain more glands than usual. In "sclerosing adenosis," or "fibrosing adenosis," the enlarged lobules are distorted by scar-like fibrous tissue. "Cysts" are abnormal sacs filled with fluid or semi-solid material, and lined by breast epithelial cells, developing from lobular structures. They begin as excess fluid inside breast glands, but may grow to proportions that stretch surrounding breast tissue, causing pain. "Fibrocysts" are cystic lesions circumscribed by, or situated within, a conspicuous amount of fibrous connective tissue. "Duct ectasia" refers to a dilation of mammary ducts by lipid and cellular debris. Rupture of the ducts induces infiltration by granulocytes and plasma cells. "Fibroadenoma" refers to benign tumors that are derived from glandular epithelium and contain a conspicuous stroma of proliferating fibroblasts and connective tissue. "Fibrosis" simply refers to a prominence of fibrous tissue in the breast. "Hyperplasia" refers to an overgrowth of cells, where several layers of cells line the basal membrane, without tumor formation. Hyperplasia increases the bulk of mammary tissue. In "epithelial hyperplasia," the cells lining breast ducts and lobules are involved, giving rise to the terms "ducal hyperplasia" and "lobular hyperplasia." Based on a histological determination, hyperplasia may be characterized as "usual" or "atypical". "Metaplasia" refers to a phenomenon in which a differentiated tissue of one type transforms into a differentiated tissue of another type. Metaplasia often results from an environmental change, and enables cells better to withstand the change;

- gynecomastia. Gynecomastia is a common clinical condition, often presenting secondarily to an underlying disorder, representing the benign and sometimes painful proliferation of breast tissue in young boys and adult males;

- breast cancer, especially non-invasive breast cancer;

- mastalgia. Mastalgia, also called "mastodynia" or breast pain, constitutes the most common breast problem for which women consult general medical practitioners. Its severity varies, but mastalgia can be so prolonged and intense as to interfere with normal daily activities, and even to disable afflicted individuals. Mastalgia can be classified according to three general sources of pain: (1) cyclical mammary pain, (2) non-cyclical mammary pain, and (3) extramammary pain. Cyclical mastalgia results from physiological breast enlargement, caused by estrogen-dependent vascular changes, during the luteal phase of the menstrual cycle, and affects a majority of premenopausal women. Cyclical mastalgia also can recur in postmenopausal women on estrogen replacement therapy, with a dose-dependent effect. "Non-cyclical mastalgia", as its name suggests, refers to pain in the breast that is not related to the menstrual cycle. A number of conditions give rise to non-cyclical mastalgia, including sclerosing adenosis, Tietz's syndrome and, rarely, breast cancer. Finally, extramammary mastalgia includes breast pain that is projected to the breast from other sources, as occurs, for example, when a patient feels pain from muscles or ribs that underlie the breasts.

- treating a patient suffering from or at risk of developing a skin disorder such as:

- pruritus, in particular cholinergic pruritus. Pruritus, or itching, can result from drug reaction, food allergy, kidney or liver disease, cancers, parasites, aging or dry skin, contact skin reaction, such as poison ivy, and for unknown reasons. In cholinergic pruritus, the symptoms are caused by sweat.

- erythema, which is a reddening of the skin caused by capillary congestion.

- urticaria. Urticaria, commonly caused by an allergic reaction, is characterized by raised red skin wheals (welts). It is also known as nettle rash or uredo. The wheals may vary in size from about 5 mm (0.2 inches) in diameter to the size of a dinner plate; they typically itch severely, sting, or burn, and often have a pale border. Urticaria is generally caused by direct contact with an allergenic substance, or an immune response to food or some other allergen, but can also appear for other reasons, notably emotional stress.

- dermatitis, in particular chronic actinic dermatitis. Chronic actinic dermatitis, also known as photosensitivity dermatitis/actinic reticuloid syndrome, is a condition in which the skin becomes inflamed, particularly in areas that have been exposed to sunlight or artificial light.

In one aspect, said skin disorder is not a subcutaneous fatty tissue disorder, in particular not cellulite.

The present pharmaceutical compositions and gels can also be used in "combination therapy" with one or more further active agent(s).

In one embodiment, the present pharmaceutical compositions and gels are particularly suitable for treating patients for whom other therapies not effective, or for treating women under hormone replacement therapy (HRT).

## Methods of Making the Compositions

The invention also provides methods for making the pharmaceutical compositions of the invention. Those skilled in the art can prepare the pharmaceutical compositions of the invention by any suitable means, based on common general knowledge. For example, danazol can be dissolved in the monoalcohol and mixed with the aqueous solvent, followed by addition of the other excipients, such as the moisturizer, and further mixing. A gelling agent, if present, can be introduced under stirring. A neutralizer, if present, usually is added at or near the end of the method, such as to the otherwise final composition. For example, if the composition comprises Carbopol, NaOH or triethanolamine can be used to neutralize the composition. Other optional components can be added at other stages of the method, in accordance with known procedures. For example, a preservative, if present, is added in an appropriate solvent, at any suitable time of the process.

## Examples

### Example 1: HPLC analysis of Danazol

HPLC Method

Danazol was analyzed by HPLC (Jasco Model PU-2080 Plus pump and Model AS-2051 Plus autosampler) using UV detection. Chromatographic resolution was obtained on a C18 reverse-phase column (5 $\mu$m, 150 mm x 4.6 mm, Dionex) with a guard column (ODS Hypersil 5 $\mu$m 10 x 4 mm, Thermo Electron Corporation, UK). The mobile phase was acetonitrile:water (65:35). The mobile phase was filtered through a 0.45 $\mu$m PVDF membrane filter. Injection volume was 50 $\mu$l and the flow rate was set at 1 ml.min-l. The UV detector (Model UV-2975 Plus) was set at 287 nm and run time was 10 min. The retention time for Danazol was 7.7 min. Each experiment was carried out in duplicate. Calibration used the external standard method. The calibration curve for Danazol was established in the range 0.1 - 20 $\mu$g.mL-1 (linearity interval for Danazol; r2 = 0.999).

Preparation of Stock Solutions

Standard solutions of danazol were prepared by dissolving 10 mg drug in 10 ml of methanol. Dilutions were made with the HPLC mobile phase. Samples were filtered through 0.45 $\mu$m PVDF membrane filters.

Assay Accuracy and Precision

Accuracy of the analytical method was calculated from the percentage of the known added amount of analyte recovered in the samples ($\pm$CV). Accuracy of the HPLC method for danazol was assessed in triplicate at two concentrations (1.0 $\mu$g.mL-1 and 20 $\mu$g.mL-1).

Accuracy of the HPLC method (i.e., recovery) for danazol was 102.08% (CV: 0.68).

Precision was measured as the degree of repeatibility of the analytical method. The precision was assessed in triplicate at two concentrations (1.0 $\mu$g.mL-1 and 20 $\mu$g.mL-1).

Repeatability CV for danazol was 0.36%.

Using this method, the limit of detection (LOD) was 30 ng/ml and the limit of quantitation (LOQ) was 100ng/ml.

Stability of Danazol in analysis medium (3% cetrimide solution)

The stability of danazol in the analysis medium (mobile phase) and receptor phase (3% cetrimide solution) was determined. At various times over 24 hours, the concentration of danazol in the sample was measured. Danazol was stable in the analysis medium and receptor phase for 30 hours.

Specificity of Method

The specificity of the method was determined. A "sham" skin diffusion experiment was performed with no drug in the donor chamber. After 24 hours, the receptor phase was sampled and subjected to HPLC analysis using the above conditions. The goal was to determine if there were any absorption peaks originating from the skin or the from the cetrimide-containing receptor phase which might interfere with the analysis of the drug. There were no other peaks observed with the same retention time as danazol.

## Example 2: Solubility Studies

The saturated solubilities of danazol were determined in a series of absolute ethanol/water mixtures (40:60, 50:50, 60:40 and 70:30, w/w) by shaking an excess amount of drug in 1 ml of the co-solvent system for at least 24 hours at room temperature. The concentrations of the saturated solutions were determined by HPLC analysis after appropriate dilution. Saturated solubilities of danazol in a series of absolute ethanol/water mixtures are set forth in Table 1.

**Table 1:** Solubility of danazol in a series of absolute ethanol/water mixtures (n=3)

| EtOH: Water (w/w) | Expt 1 ($\mu$g.ml-1) | Expt 2 ($\mu$g.ml-1) | Expt 3 ($\mu$g.ml-1) | Average concentration$\pm$SD ($\mu$g.ml-1) |
|---|---|---|---|---|
| 40:60 | 388 | 393 | 378 | 386$\pm$8 8 |
| 50:50 | 1424 | 1329 | 1325 | 1359$\pm$56 |
| 60:40 | 3189 | 3379 | 3479 | 3349$\pm$147 |
| 70:30 | 7774 | 6940 | 7483 | 7399$\pm$423 |

The solubility of danazol in such hydroalcoholic formulations is increased by increasing the percentage of alcohol.

The solubility of drug was also determined in the 3% cetrimide solution. The solubility of danazol in 3% cetrimide solution was determined to be 2113 $\pm$ 15 $\mu$g/mL.

## Example 3: *In silico* prediction of skin permeation

Transdermal delivery of:

- danazol and
- the structurally related compound testosterone

were estimated *in silico* by calculating a maximum theoretical flux ($J_{max}$) across skin according to the following approach:

The maximum flux ($J_{max}$) at which a chemical can cross the skin is theoretically achieved when it is maintained in a saturated solution (or in neat chemical form) on the surface. The relevant equation that applies in these circumstances is Fick's 1st law:

$$J_{max} \cong \frac{D}{h} * K_{skin/vehicle} * C_{vehicle}^{sat} \qquad \textbf{(Equation 1)}$$

where $D$ is the chemical's diffusivity across the skin (topically that through the stratum corneum, the skin's least permeable and outermost layer), $h$ is the diffusion path-length, $K_{skin/vehicle}$ is the compound's partition coefficient between the skin and the vehicle contacting the surface, and $C_{vehicle}^{sat}$ is its saturation solubility in the vehicle. $K_{skin/vehicle}$ may be defined as follows:

$$K_{skin/vehicle} = \frac{C_{skin}^{sat}}{C_{vehicle}^{sat}} \qquad \textbf{(Equation 2)}$$

where $C_{skin}^{sat}$ and $C_{vehicle}^{sat}$ are the concentrations in skin and vehicle respectively. Equation 1 can then be reduced to a simpler form

$$J_{max} = \frac{D}{h} * C_{skin}^{sat}$$   **(Equation 3)**

which shows that the maximum flux achievable across the barrier is independent of the formulation, providing that the formulation is saturated. That is, $J_{max}$ should be constant as long as the chemical is at its maximum thermodynamic activity in the vehicle (i.e., that it is saturated), and provided that the excipients in the formulation do not change the skin's barrier properties (e.g., exhibit permeation-enhancing or -retarding characteristics).

Hence, if the value of $J_{max}$ can be predicted from first principles, it should then be possible, with knowledge of the degree of saturation of the chemical in a particular formulation, to calculate the maximum amount absorbed across the skin following a specific scenario.

This objective can be achieved using an algorithm derived by Potts & Guy (Pharm Res. 1992, 9(5), 663-9) from an extensive database of the permeability coefficients of approximately 100 chemicals across human skin *in vitro* following their application in water. The permeability coefficient of a chemical ($K_p$) from an aqueous vehicle is defined as:

$$K_p = \frac{D * K_{skin/water}}{h}$$   **(Equation 4)**

Multiple regression analysis of the experimental values of $K_p$ against various physicochemical variables led to the derivation of an equation (Potts & Guy 1992), which has been shown to have reasonable predictive power:

$$\log K_p = -2.7 + 0.71 * \log P - 0.0061 * MW$$   **(Equation 5)**

where $P$ is the octanol-water partition coefficient of the chemical and $MW$ is its molecular weight.
In the above form, the units of permeability coefficient are cm/h. For very lipophilic chemicals, it is necessary to correct the value of $K_p$ calculated from Equation 5 to take into account the contribution of the living skin layers (viable epidermis and dermis) to the permeation process (Cleek and Bunge, Pharm Res, 1993, 10(4), 497-506):

$$K_p^{corr} = \frac{K_p}{1 + \frac{K_p \cdot \sqrt{MW}}{2.6}}$$   **(Equation 6)**

Combining Equations 1, 2 and 6 yields:

$$J_{max} = K_p * C_{water}^{sat}$$   **(Equation 7)**

The permeability coefficient can be calculated from Equations 5 and 6 and readily available physicochemical parameters ($MW$ and $log P$), for which a very large database of values exists, or which can be calculated with many different approaches available on the internet. Equally, there is a considerable number of aqueous solubilities tabulated and/or accessible via the web.

Aqueous solubility ($C_{water}^{sat}$) and the partition coefficient between octanol and water (logP) of danazol and testosterone were estimated using the Osiris Property Explorer calculator (from Actelion's inhouse registration system). (http://www.chemexper.com).

According to this calculator, properties of a given molecule are predicted by a fragment-based approach by comparison with molecules for which those properties have been determined experimentally (see Balakin et al., Current Medicinal Chemistry, 2006, 13, p223-241, "In Silico approaches to prediction of aqueous and DMSO solubility of drug-like compounds: trends, problems and solutions" for a review on the in silico prediction of aqueous solubility of drug-like compounds).

The following results were obtained:

Table 2: Predicted transdermal flux for testosterone and danazol using the Osiris property explorer estimates

| Compound | MW | Osiris predictions | | Predicted Jmax ($\mu$g/cm$^2$/h) |
|---|---|---|---|---|
| | | logP | $C_{water}^{sat}$ | |
| Testosterone | 288.4 | 3.58 | 2.50E-02 | 0.13 |
| Danazol | 337.5 | 3.13 | 4.45E-03 | 0.014 |

The predicted maximum flux for testosterone was 0.13 $\mu$g/cm$^2$/h, which is similar to experimentally determined values, which range from 0.13 to 0.2 $\mu$g/cm$^2$/h (see http://edetox.ncl.ac.uk/searchinvitro.aspx).

As mentioned above, testosterone and danazol are structurally closely related molecules. Consequently, finding that the *in silico* predictions presented above are accurate in the case of testosterone, the skilled person would expect the predictions to be accurate in the case of danazol as well.

The predicted maximum flux for danazol according to this model was 0.014 $\mu$g/cm$^2$/h, which is approximately ten times lower than that of testosterone. Therefore, the skilled person would be strongly dissuaded from envisaging a transdermal delivery route for administering danazol, since he/she would have expected poor results, and in particular poor flux values. Thus, the *in silico* prediction severely undermines the feasibility of transdermal treatment with danazol.

### Example 4: Skin Permeation Study (Franz cells)

The permeation studies were carried out using opaque side-by-side diffusion cells with an effective diffusion area of 0.71 cm$^2$. The receptor compartment had a volume of 3.2 ml and was maintained at 37$\pm$0.5°C. The receptor solution was a 3% w/v cetrimide solution and allowed sink conditions to be maintained. The receptor phase was magnetically stirred at 100 rpm. Dermatomed abdominal pig skin (750 $\mu$m) was used. Skin was allowed to hydrate with isotonic saline solution for 1 h before the experiment was started. At t = 0, 3 ml of a saturated solution of Danazol in 70:30 w/w ethanol/water was introduced into the donor chamber. Experiments were performed under complete occlusion. Post-application, at scheduled times (3, 6, 9, 20 and 24 hours), 1 ml samples were taken from the receiver compartment and replaced with the same volume of fresh, temperature-equilibrated receptor fluid. Samples were filtered through 0.45 $\mu$m PVDF membrane filters. The amount of Danazol in the samples was determined by HPLC using the method previously described.

Table 3: Cumulative permeation ($\mu$g.cm$^{-2}$) of danazol through abdominal pig skin from a saturated solution of the drug in 70:30 w/w ethanol-water.

| Time (hr) | Cell | Cell 2 | Cell 3 | Cell 4 | Cell 5 | Cell 6 | Cell 7 | Cell 8 | **Mean $\pm$ SD** |
|---|---|---|---|---|---|---|---|---|---|
| 3 | - | - | - | - | nd$ | - | - | - | |
| 6 | - | - | - | - | nd$ | - | - | - | |
| 9 | - | - | - | - | nd$ | - | 1.1 | - | |
| 20 | 1.2 | 1.8 | 0.8 | 0.7 | nd$ | 1.5 | 12.1 | 0.9 | **2.7$\pm$4.2** |
| 24 | 2.0 | -* | 1.2 | 1.2 | 20.3$ | 3.1 | 20.5 | 1.7 | **7.2$\pm$9.1** |

-= the quantity was below the limit of detection (LOD) of 30ng/ml.
nd = not determined
* Cell was leaking at 24 h; value obtained was 'off-scale'.
$ Only a single sample was taken at 24 h.

Therefore, the experimentally determined maximum flux observed for danazol was around 7.2/24 = 0.3 $\mu$g/cm$^2$/h. This value is comparable to that observed for testosterone (both *in silico* and *in vitro*) and is one order of magnitude higher than that predicted by the *in silico* calculations.

**Table 4**: Predicted vs. experimental transdermal flux for testosterone and danazol using the Osiris property explorer estimates

| Compound | MW | Osiris predictions | | Jmax ($\mu$g/cm$^2$/h) | |
|---|---|---|---|---|---|
| | | logP | $C^{sat}_{water}$ | predicted | experimental |
| Testosterone | 288.4 | 3.58 | 2.50E-02 | 0.13 | 0.13 to 0.2 |
| Danazol | 337.5 | 3.13 | 4.45E-03 | **0.014** | **0.3** |

Thus, the applicant has surprisingly determined that transdermal delivery of danazol using a composition according to the invention is indeed possible, contrary to the *in silico* predictions.

The results reported above indicate that the compositions of the invention are advantageously suitable for topical administration to a skin surface, for effective treatment.

Based on the bioavailability of oral danazol (11%), and an oral therapeutic oral dose of 100 mg, the amount of danazol providing therapeutic effect is 11 mg.

Based on the flex observed above (7.2 $\mu$g/cm$^2$ over 24 hours), a topical composition could deliver 11 mg over a surface of 1500cm$^2$, which could be achieved in a maximum of 2 applications per breast.

**Claims**

1. A pharmaceutical composition for topical administration to a skin surface wherein said composition comprises:

    (i) a therapeutically effective amount of danazol,
    (ii) at least one monoalcohol,
    (iii) optionally, a non-irritating and/or non-sensitizing amount of at least one penetration enhancer,
    (iv) optionally, at least one gelling agent,
    (v) optionally, at least one moisturizer,
    (vi) an aqueous vehicle.

2. A pharmaceutical composition according to claim 1 wherein said composition comprises:

    (i) 0.01 to 10 % (w/w) of danazol,
    (ii) 10 to 90 % (w/w) of at least one monoalcohol, e.g., ethanol or isopropanol,
    (iii) 0 to 10 % (w/w) of at least one penetration enhancer, e.g., isopropyl myristate or propylene glycol,
    (iv) 0 to 5 % (w/w) of at least one gelling agent, e.g., polyacrylic acids, cellulosics, or mixtures thereof,
    (v) 0 to 30 % (w/w) of at least one moisturizer, e.g., glycerine,
    (vi) q.s. 100 % (w/w) water.

3. A pharmaceutical composition according to claim 1 or 2, for treating a patient suffering from or at risk of developing a breast disorder selected from benign breast diseases, gynecomastia, breast cancer, mastalgia and conditions involving dense breast tissue.

4. A pharmaceutical composition according to claim 1 or 2, for treating a patient suffering from or at risk of developing a skin disorder selected from pruritus, erythema, urticaria, and dermatitis.

5. Use of danazol in the preparation of a pharmaceutical composition according to claim 1 or 2, wherein said pharmaceutical composition is for treating a patient suffering from or at risk of developing a breast disorder selected from benign breast diseases, gynecomastia, breast cancer, mastalgia and conditions involving dense breast tissue.

6. Use of danazol in the preparation of a pharmaceutical composition according to claim 1 or 2, wherein said pharmaceutical composition is for treating a patient suffering from or at risk of developing a skin disorder selected from pruritus, erythema, urticaria, and dermatitis.

7. Dose packet, unit dose packet or multiple dose packet containing a pharmaceutical composition according to claim 1 or 2.

8. Dispenser, optionally. with hand pump, containing a pharmaceutical composition according to claim 1 or 2.

9. Device comprising:

   (A) a reservoir containing a pharmaceutical composition comprising:

      (i) a therapeutically effective amount of danazol,
      (ii) at least one monoalcohol,
      (iii) optionally, a non-irritating and/or non sensitizing amount of at least one penetration enhancer,
      (iv) optionally, at least one gelling agent,
      (v) optionally, at least one moisturizer, and

   (B) a topical applicator.

10. Process for preparing a pharmaceutical composition according to any one of claims 1 or 2 comprising the steps of preparing a mixture comprising:

      (i) at least one monoalcohol,
      (ii) danazol,
      (iii) an aqueous vehicle,
      (iv) optionally, a non-irritating and/or non-sensitizing amount of at least one penetration enhancer,
      (v) optionally, at least one gelling agent,
      (vi) optionally, at least one moisturizer.

11. Method of administering a therapeutically effective amount of danazol to a patient in need thereof, comprising topically administering to a surface of skin of said patient a pharmaceutical composition according to claim 1 or 2

12. Method for treating a patient suffering from or at risk of developing a breast disorder comprising topically administering to a surface of skin of a patient in need thereof a therapeutically effective amount of a pharmaceutical composition according to claim 1 or 2.

13. Method for treating a patient suffering from or at risk of developing a skin disorder comprising topically administering to a surface of skin of a patient in need thereof a therapeutically effective amount of a pharmaceutical composition according to claim 1 or 2.

Europäisches
Patentamt

European
Patent Office

Office européen
des brevets

## EUROPEAN SEARCH REPORT

**Application Number**

EP 08 30 5421

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/002868 A1 (GONDA IGOR [AU] ET AL) 6 January 2005 (2005-01-06) * page 1, paragraph 1 * * page 2, paragraph 28-30 * * page 3, paragraph 44-50 * * page 4, paragraph 64-66 * ----- | 1-13 | INV. A61K31/58 A61P17/04 A61P35/00 |
| X | WO 02/087543 A (BIOZONE LAB INC [US]) 7 November 2002 (2002-11-07) * page 10; example 7 * ----- | 1-13 | |
| X | US 2005/003009 A1 (FRANKE PATRICK [DE]) 6 January 2005 (2005-01-06) * page 4, paragraph 30 * * page 6, paragraphs 54,55 * * page 7, paragraphs 73,85 * * page 8, paragraph 96 - page 9, paragraph 115 * ----- | 1-13 | |
| X | WO 2005/039531 A (ANTARES PHARMA IPL AG [CH]) 6 May 2005 (2005-05-06) * page 13, lines 6-23 * * page 16, line 4 * ----- | 1-13 | TECHNICAL FIELDS SEARCHED (IPC) A61K A61P |
| X | AU 2006 207 868 A1 (ADVANCED INHALATION RES INC) 28 September 2006 (2006-09-28) * page 31, paragraph 3 * ----- | 1-8,10 | |
|  | -/-- | | |

The present search report has been drawn up for all claims

| Place of search | Date of completion of the search | Examiner |
|---|---|---|
| The Hague | 19 November 2008 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (IPC) |
| X | BRUSEWITZ ET AL: "Novel poloxamer-based nanoemulsions to enhance the intestinal absorption of active compounds" INTERNATIONAL JOURNAL OF PHARMACEUTICS, ELSEVIER BV, NL, vol. 329, no. 1-2, 20 December 2006 (2006-12-20), pages 173-181, XP005809189 ISSN: 0378-5173 * page 178, left-hand column, last paragraph; table 3 * ----- | 1-8,10 | |
| D,X<br>A | WO 2004/060322 A (FEMMEPHARMA INC [US]) 22 July 2004 (2004-07-22) * page 8, lines 3-11 *<br><br>* page 8, line 25 - page 9, line 24 * * page 10, lines 10-32 * ----- | 9<br><br>1-8,<br>10-13 | |
| A | NORMAN R A: "CAUSES AND MANAGEMENT OF XEROSIS AND PRURITIS IN THE ELDERLY" ANNALS OF LONG-TERM CARE, MULTIMEDIA HEALTHCARE, PLAINSBORO, US, vol. 9, no. 12, 1 December 2001 (2001-12-01), pages 35-40, XP001119395 ISSN: 1524-7929 * the whole document * ----- | 1-13 | TECHNICAL FIELDS SEARCHED (IPC) |

The present search report has been drawn up for all claims

| Place of search | Date of completion of the search | Examiner |
|---|---|---|
| The Hague | 19 November 2008 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 30 5421

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2008

| Patent document cited in search report | | Publication date | Patent family member(s) | | Publication date |
|---|---|---|---|---|---|
| US 2005002868 | A1 | 06-01-2005 | CA | 2433101 A1 | 23-12-2004 |
| WO 02087543 | A | 07-11-2002 | NONE | | |
| US 2005003009 | A1 | 06-01-2005 | NONE | | |
| WO 2005039531 | A | 06-05-2005 | AU | 2004283431 A1 | 06-05-2005 |
| | | | BR | PI0414551 A | 31-10-2006 |
| | | | CA | 2538856 A1 | 06-05-2005 |
| | | | EP | 1670433 A1 | 21-06-2006 |
| | | | JP | 2007508261 T | 05-04-2007 |
| | | | MX | PA06003316 A | 08-06-2006 |
| | | | US | 2006153905 A1 | 13-07-2006 |
| AU 2006207868 | A1 | 28-09-2006 | NONE | | |
| WO 2004060322 | A | 22-07-2004 | AU | 2004203700 A1 | 22-07-2004 |
| | | | CA | 2511995 A1 | 22-07-2004 |
| | | | EP | 1578421 A2 | 28-09-2005 |
| | | | JP | 2006515026 T | 18-05-2006 |
| | | | MX | PA05007266 A | 17-01-2006 |

For more details about this annex : see Official Journal of the European Patent Office, No. 12/82

**EP 2 147 674 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004060322 A
- WO 9527569 A
- WO 9615045 A
- US 5630531 A

### Non-patent literature cited in the description

- **Wong A.Y. ; Tang L.** *Fertil Steril,* 2004, vol. 81 (6), 1522-7
- **Potts ; Guy.** *Pharm Res.,* 1992, vol. 9 (5), 663-9
- **Cleek ; Bunge.** *Pharm Res,* 1993, vol. 10 (4), 497-506
- **Balakin et al.** In Silico approaches to prediction of aqueous and DMSO solubility of drug-like compounds: trends, problems and solutions. *Current Medicinal Chemistry,* 2006, vol. 13, 223-241